# EUROPEAN PATENT SPECIFICATION

(11) **EP 4 611 601 B1**
(45) Date of publication and mention of the grant of the patent: **02.09.2026**
(21) Application number: 23809916.2
(22) Date of filing: 26.10.2023
(51) Int. Cl.: A61B 1/12, A61B 90/70

(54) **DRAIN SYSTEM FOR CENTRIFUGAL PUMP OPERATED ENDOSCOPE REPROCESSOR**
ABFLUSSSYSTEM FÜR EINEN ZENTRIFUGALPUMPENBETRIEBENEN ENDOSKOPREPROZESSOR
SYSTÈME DE DRAINAGE POUR DISPOSITIF DE RETRAITEMENT D'ENDOSCOPE ACTIONNÉ PAR POMPE CENTRIFUGE

(30) Priority: 04.11.2022 US 202263422459 P
(43) Date of publication of application: 10.09.2025
(73) Proprietor: American Sterilizer Company, Mentor, OH 44060 (US)
(72) Inventor: GETSY, Andrew Paul, Kirtland, Ohio 44094 (US)
(74) Representative: FRKelly
(86) International application number: PCT/US2023/077820
(87) International publication number: WO 2024/097570

(56) References cited:
- JP-A- 2009 142 324
- KR-A- 20200 069 793
- US-A1- 2009 050 181
- US-A1- 2014 134 071

## Description

### Field of Invention

This application relates generally to a drain system for an endoscope reprocessor operated by a centrifugal pump, and more particularly to a drain system that drains fluid with the presence of air therein more efficiently from a basin of the centrifugal pump operated endoscope reprocessor.

### Background

Medical device processors, also referred to as medical instrument processors, are widely used in various health care settings and are typically associated with a sterilization claim or a high level disinfection claim. An example of such a processor is an automated endoscope reprocessor (AER) used for reprocessing endoscopes, such as duodenoscopes, and endoscope accessories. AERs are designed to kill microorganisms in or on reusable endoscopes by exposing their outside surfaces and interior channels to liquid chemical sterilant or high level disinfectant solutions.

One type of endoscope reprocessor includes a cleaning basin into which the endoscope is placed and a centrifugal pump that introduces fluid into the basin including the endoscope therein for decontamination processing. Air usually is introduced into the basin during the processing cycle whether by design or as an effect in decontaminating the medical instrument. A drain is provided to drain the basin during and after the processing cycle. A drain screen is provided at the surface of an inlet of the drain, flush with the bottom of the basin, to capture debris in the fluid. US 2014/134071 A1 discloses an endoscope reprocessor comprising a lid displaceable between an open position and a sealed position to seal the top opening, and a drain having an inlet at the perimeter of the bottom opening configured to drain fluid exiting from the basin. A pump is configured to circulate fluid to and from the basin via the drain when the lid is in the sealed position. US 2009/050181 A1 discloses a reprocessing device for medical instruments comprising a centrifugal pump configured to pump fluid to a basin and from the basin via a drain. The centrifugal pump is used to circulate processing fluid through the device during a reprocessing cycle.

For drain systems or mechanisms in some centrifugal pump operated endoscope reprocessors, there remain various shortcomings, drawbacks, and disadvantages relative to certain applications. The drain systems of the afore mentioned centrifugal pump operated reprocessors, for example, face challenges in managing air that is introduced into the fluid during a processing cycle.

Accordingly, there remains a need for further contributions in this area of technology.

### Summary of Invention

The invention is defined by the features of independent claim 1. Preferred embodiments are defined in the dependent claims 2-19. According to the claimed invention, a centrifugal pump operated endoscope reprocessor includes a basin having a top opening for receiving therein a medical device for processing and a bottom opening from which fluid exits the basin; a lid displaceable relative to the basin between an open position to expose the top opening and a sealed position to seal the top opening; a drain having an inlet at the perimeter of the bottom opening of the basin and being configured to drain fluid exiting from the bottom opening, wherein the inlet has an inner diameter; a centrifugal pump configured to pump fluid to the basin via one or more passageways with the lid in the sealed position and from the basin via the drain, wherein the drain extends downward from the perimeter of the bottom opening of the basin to a fluid line leading to the centrifugal pump, wherein the drain defines a drain axis along which the fluid flows as the centrifugal pump pumps fluid from the basin; and, a drain screen configured to capture debris in the fluid, wherein the drain screen is positioned in the drain transverse to the drain axis at a depth distance from the inlet of the drain in the range of about 50 percent to about 200 percent of the inner diameter, and wherein the drain screen has an open area percent in the range of greater than 0 percent and up to about 70 percent of a drain area of the drain at the position of the drain screen in the drain.

Embodiments of the invention may include one or more of the following additional features separately or in combination.

The depth distance may be about 75 percent of the inner diameter.

The centrifugal pump may have a pump open area that is about the same area as the open area of the drain screen.

The drain screen may include a perforated plate.

The perforated plate may have a plurality of perforations having a widest width less than 6.35 mm (1/4 inch).

The widest width of the perforations may be less than or equal to 4.76 mm (3/16 inch).

The drain screen may comprise an insert removable from the drain.

The centrifugal pump operated endoscope reprocessor may include a sanitary gasket.

The sanitary gasket may be positioned in the drain at the same depth distance from the inlet of the drain as the drain screen.

An outer periphery of the drain screen may be molded in an inner periphery of the sanitary gasket.

A bottom wall of the basin may have a negative slope relative to horizontal in the direction toward the bottom opening, and the bottom opening may be positioned at the deepest part of the basin.

The centrifugal pump operated endoscope reprocessor may include a spray mechanism configured to spray jets of fluid within the basin.

The spray mechanism may include a spray bar rotatably mounted to an underside of the lid.

The centrifugal pump operated endoscope reprocessor may include a compressed air source configured to introduce compressed air into the basin as fluid is drained from the basin via the drain.

According to another aspect of the invention, a method of processing a medical device in a centrifugal pump operated endoscope reprocessor is provided, the centrifugal pump operated endoscope reprocessor including a basin having a top opening, a bottom opening from which fluid exits the basin, a lid, a centrifugal pump, and a drain, the drain having an inlet at the perimeter of the bottom opening and being configured to drain fluid exiting from the bottom opening, the inlet having an inner diameter, and wherein the drain extends downward from the perimeter of the bottom opening to a fluid line leading to the centrifugal pump and the drain defines a drain axis along which the fluid flows as the centrifugal pump pumps fluid from the basin, the method including inserting a medical device through the top opening into the basin; displacing the lid from an open position to a sealed position to seal the top opening; conducting a decontamination cycle including pumping by the centrifugal pump decontaminant fluid into the basin via one ore more passageways with the lid in the sealed position and from the basin via the drain; introducing air into the basin; using a drain screen to capture debris in the fluid, wherein the drain screen is positioned in the drain transverse to the drain axis at a depth distance from the inlet of the drain in the range of about 50 percent to about 200 percent of the inner diameter, and wherein the drain screen has an open area percent in the range of greater than 0 percent and up to about 70 percent of a drain area of the drain at the position of the drain screen in the drain.

Embodiments of the invention may include one or more of the following additional features separately or in combination.

The method may include positioning a sanitary gasket in the drain at the same depth distance from the inlet of the drain as the drain screen.

The method may include introducing air into the basin includes spraying jets of fluid within the basin.

The method may include introducing air into the basin includes using a compressed air source to introduce air into the basin as fluid is drained from the basin via the drain.

The method may include inserting the medical device includes inserting an endoscope such that a handle of the endoscope is positioned at the surface of the drain inlet.

The following description and the annexed drawings set forth certain illustrative embodiments of the invention. These embodiments are indicative, however, of but a few of the various ways in which the principles of the invention may be employed. Other objects, advantages and novel features according to aspects of the invention will become apparent from the following detailed description when considered in conjunction with the drawings.

### Brief Description of the Drawings

The annexed drawings, which are not necessarily to scale, show various aspects of the invention.
Fig. 1 is a perspective view of a centrifugal pump operated endoscope reprocessor according to an embodiment of the invention with a lid thereof in an open position.
Fig. 2 is a perspective view of the Fig. 1 reprocessor shown from the left and with the lid omitted to show a bottom opening and a drain of the reprocessor.
Fig. 3 is top plan view of the Fig. 1 processor, showing an endoscope positioned in a basin of the reprocessor.
Fig. 4 is a left side cross sectional view of the Fig. 1 processor, showing the basin, the drain, and a centrifugal pump of the reprocessor.
Fig. 5 is an enlarged view of the drain of the Fig. 1 reprocessor.
Fig. 6 is an enlarged perspective view of a drain screen of the Fig. 1 reprocessor.
Fig. 7 is an enlarged perspective view of a drain screen according to an embodiment of the invention.
Fig. 8 shows a flowchart of a method in accordance with an embodiment of the invention.

### Detailed Description

While the present invention can take many different forms, for the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications of the described embodiments, and any further applications of the principles of the invention as described herein, are contemplated as would normally occur to one skilled in the art to which the invention relates.

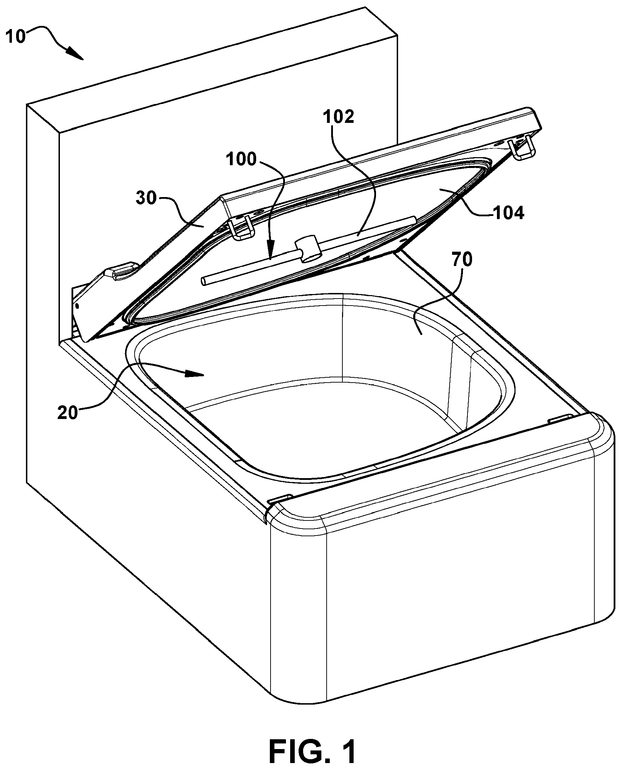

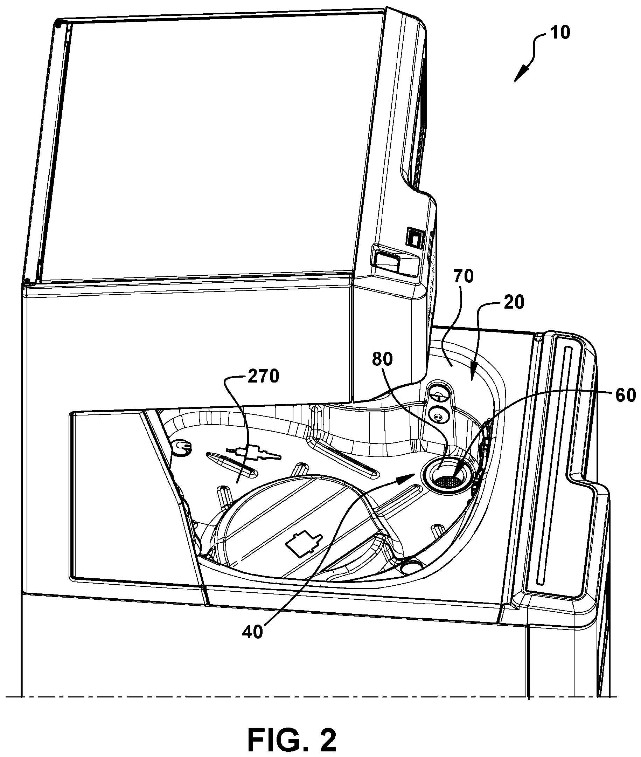

Figs. 1-7 show a centrifugal pump operated endoscope reprocessor 10 in accordance with an embodiment of the invention. The centrifugal pump operated endoscope reprocessor 10 may be any type of system for processing medical devices or instruments, for example, by a sterilization process or a disinfection process. In the illustrated embodiment, the centrifugal pump operated endoscope reprocessor 10 comprises an automated endoscope reprocessor (AER) 10 configured to reprocess endoscopes such as duodenoscopes, and endoscope accessories. The centrifugal pump operated endoscope reprocessor 10 includes a basin 20, a lid 30, a drain 40, a centrifugal pump 50, and a drain screen 60, 300. The basin 20 has a top opening 70 for receiving therein a medical device such as an endoscope 72 for processing and a bottom opening 74 from which fluid exits the basin 20. The lid 30 is displaceable relative to the basin 20 between an open position to expose the top opening 70, see Fig. 1, and a sealed position to seal the top opening 70. The drain 40 has an inlet 80 at the perimeter of the bottom opening 74 of the basin 20 and is configured to drain fluid exiting from the bottom opening 74. The inlet 80 has an inner diameter D1. The centrifugal pump 50 is configured to pump fluid to the basin 20 via one ore more passageways with the lid 30 in the sealed position and from the basin 20 via the drain 40. The drain 40 extends downward from the perimeter of the bottom opening 74 of the basin 20 to a fluid line 90 leading to the centrifugal pump 50. The drain 40 defines a drain axis A-A along which the fluid flows as the centrifugal pump 50 pumps fluid from the basin 20. The drain screen 60, 300 is configured to capture debris in the fluid and is positioned in the drain 40 transverse to the drain axis A-A at a depth distance D2 from the inlet 80 of the drain 40 in the range of about 50 percent to about 200 percent of the inner diameter D1 of the inlet 80. The drain screen 60, 300 has an open area percent in the range of greater than 0 percent and up to about 70 percent of a drain area of the drain 40 at the position of the drain screen 60, 300 in the drain 40.

As will be described in greater detail below, the drain screen 60, 300 being positioned at the depth distance D2 and configured with the open area percent that is greater than 0 percent and up to about 70 percent allows the centrifugal pump operated endoscope reprocessor 10 to drain fluid from the basin 20 even with the presence of air in the fluid and presents the fluid and air to the centrifugal pump 50 in a consumable way. Advantageously, the drain screen 60, 300 improves drain time of the fluid from the drain 40 and prevents cavitation of the centrifugal pump 50.

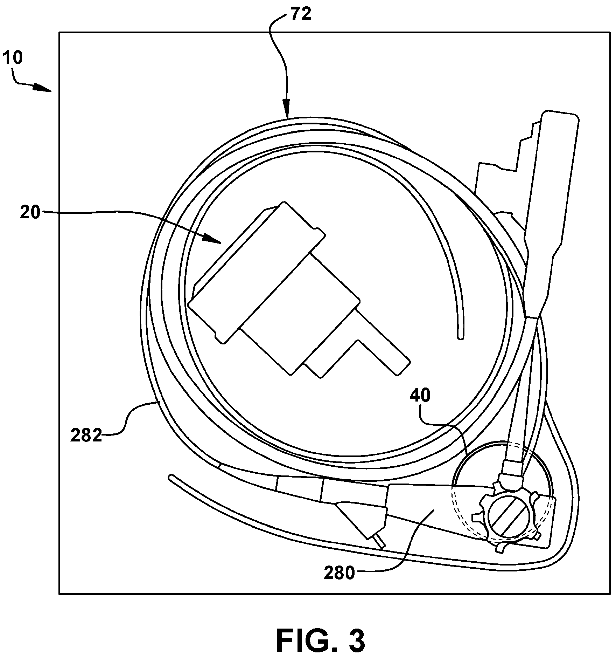

Turning initially then to Figs. 1-4, the lid 30 of the centrifugal pump operated endoscope reprocessor 10 is configured for angular displacement between the open position and the sealed position. Any suitable mechanism or combination of mechanisms may be used to displace the lid 30 between the open and sealed positions. In operation, when in the open position shown in Fig. 1, medical devices such as an endoscope can be inserted into the basin 20 of the centrifugal pump operated endoscope reprocessor 10, as shown in Fig. 3. The lid 30 can then be sealed and thereafter a processing cycle, also referred to herein as a reprocessing cycle, can then be performed by the centrifugal pump operated endoscope reprocessor 10. The illustrated centrifugal pump operated endoscope reprocessor 10 is configured, for example, to introduce a decontaminant fluid such as a liquid chemical sterilant or high level disinfectant solution into the basin 20 to expose the outside surfaces and interior channels of the endoscope to the fluid. Once the processing cycle is completed, the order of operation is merely reversed. The lid 30 is displaced from the sealed position to the open position and the processed medical device can then be removed from the basin 20.

As shown in Fig. 1, the centrifugal pump operated endoscope reprocessor 10 may include a spray mechanism 100 configured to spray jets of fluid within the basin 20 during a processing cycle. In the illustrated embodiment, the spray mechanism 100 includes a spray bar 102 rotatably mounted to an underside 104 of the lid 30. As the spray bar 102 rotates during a processing cycle, the spray bar 102 sprays jets of fluid down into the fluid in the basin 20 and onto the surfaces of the basin 20 and the surfaces of the endoscope 72. As will be described in greater detail below, spraying the jets of fluid into the fluid in the basin 20 may have the effect of introducing air in the basin 20 and the fluid therein during the processing cycle.

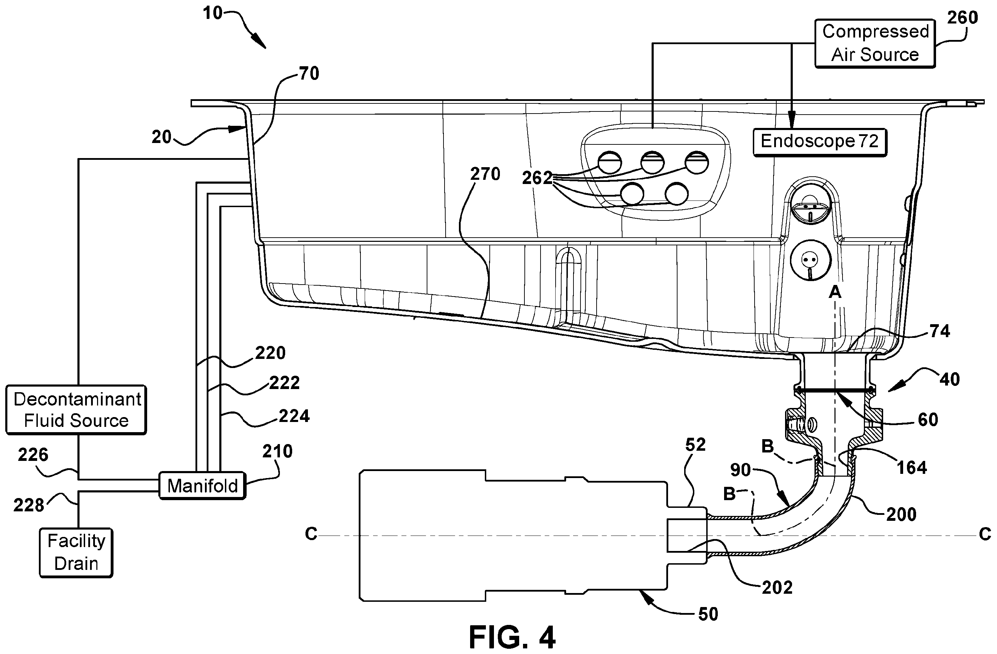

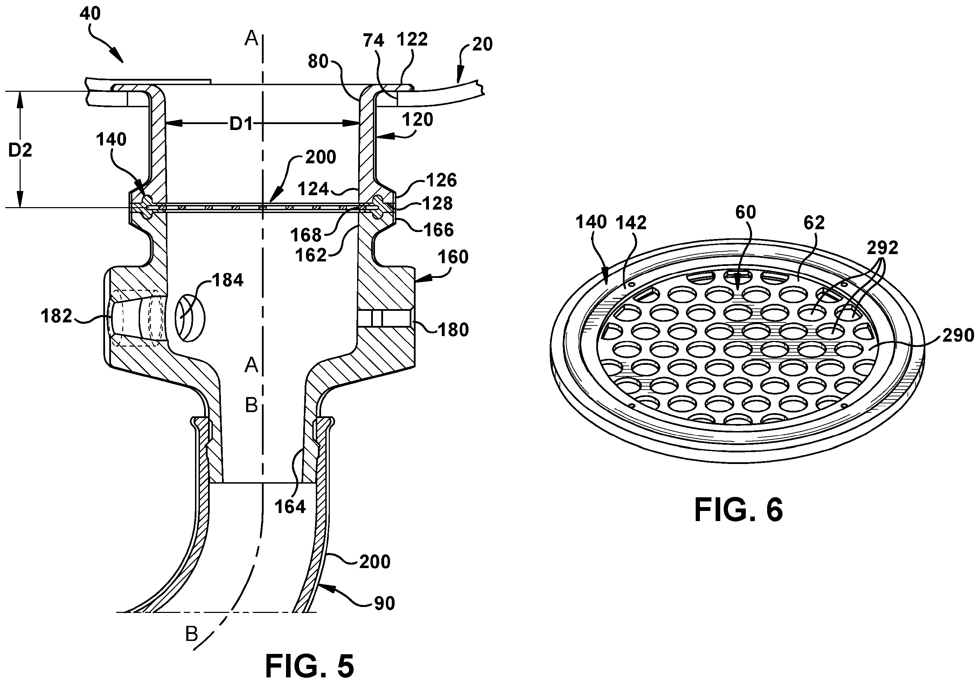

Referring to Figs. 4 and 5, the drain 40 of the centrifugal pump operated endoscope reprocessor 10 may include an adapter piece 120, a sanitary gasket 140, and a port manifold 160, disposed in this order along the drain axis A-A. In the illustrated embodiment, the drain axis A-A is oriented vertically, for example, at right angles to a top surface of the drain inlet 80, where the top surface of the drain inlet 80 lies in a horizontal plane in the illustrated embodiment. As used herein, horizontal means the plane of the horizon, and vertical means at a right angle, or perpendicular, to the horizontal or horizon. It will be appreciated that the drain axis A-A may be a curved axis that changes from a vertical orientation to a non-vertical orientation, and that any one or more of the adapter piece 120, the sanitary gasket 140, and the port manifold 160 may be arranged along the curved axis such that a central axis of one is oriented at an angle relative to a central axis of another.

The adapter piece 120 is positioned adjacent and vertically below the drain inlet 80. Further, the adapter piece 120 is configured to provide an unimpeded flow passage from the inlet 80 to the drain screen 60. In the illustrated embodiment, the unimpeded flow passage is oriented vertically in the direction of the drain axis A-A such that the flow passage in cross section view looking down the axis A-A has a round shape area. In the illustrated embodiment, the unimpeded flow passage is in the form of a rigid tube-like member having the inner diameter D1, that is, the same as the inner diameter D1 of the drain inlet 80. The round or tube-like geometry, in contrast to a non-round geometry, aids in less friction and increased flow and efficiency through the adapter piece 120 and thus through the drain 40. The inner diameter D1 of the tube-like member may be constant or non-constant. In the Fig. 5 embodiment, the inner diameter D1 of the tube-like member gets slightly smaller as the depth from the inlet 80 increases owing to a draft angle necessary to make the adapter piece 120 as an injection molded part. It is contemplated that the inner diameter D1 of the tube-like member may change slightly more or slightly less so long as the open area percent of the drain screen 60 corresponding to the inner diameter is in the afore mentioned range of greater than 0 percent and up to about 70 percent.

The sanitary gasket 140 and an upstream portion of the port manifold 160 also have a tube-like geometry and have centerlines that coincide with the centerline of the adapter piece 120; thus, the centerlines of the adapter piece 120, the sanitary gasket 140, and the port manifold 160 also coincide with the drain axis A-A. In the illustrated embodiment, the inner diameter of the sanitary gasket 140 and the inner diameter of the upstream portion of the port manifold 160 is the same as the inner diameter of the adapter piece 120.

An upstream end 122 of the adapter piece 120 is glued or otherwise sealingly connected to the basin 20 to put the bottom opening 74 of the basin 20 in fluid communication with the adapter piece 120. An opposite or downstream end 124 of the adapter piece 120 has a sanitary connection geometry, as does an upstream end 162 of the port manifold 160. The sanitary gasket 140 creates a watertight connection between the downstream end 124 of the adapter piece 120 and the upstream end 162 of the port manifold 160. In the illustrative embodiment, the downstream end 124 of the adapter piece 120 and the upstream end 162 of the port manifold 160 are each configured with respective flanges 126, 166. To create the watertight connection the two flanges 126, 166 are mated together with the sanitary gasket 140 sandwiched between opposing faces 128, 168 of the flanges 126, 166, and a tri-clamp is used to secure the connection in place. As will be appreciated, the tri-clamp fitting allows the sanitary gasket 140 to be removed easily for cleaning and replacement if necessary.

As shown in Figs. 4 and 5, in the illustrated embodiment the sanitary gasket 140 is positioned in the drain 40 at the same depth distance D2 from the inlet 80 of the drain 40 as the drain screen 60. Also, as shown in Fig. 6, in the illustrated embodiment an outer periphery 62 of the drain screen 60 is molded in an inner periphery 142 of the sanitary gasket 140. Thus, the sanitary gasket 140 functions as an insert of the drain screen 60, making the drain screen 60 compatible with a tri-clamp fitting as described above, and thus enabling the drain screen 60 to be removed easily for cleaning and replacement if necessary. Both the sanitary gasket 140 and the drain screen 60 are positioned between the downstream end 124 of the adapter piece 120 and the upstream end 162 of the port manifold 160.

As will be appreciated, the sanitary gasket 140 need not be positioned in the drain 40 at the same depth distance D2 from the inlet 80 of the drain 40 as the drain screen 60, and other embodiments are contemplated. Thus, the sanitary gasket 140 may be positioned at a depth distance from the inlet 80 that is different from the depth distance D2 of the drain screen 60 from the inlet 80. In some embodiments, the sanitary gasket 140 may be positioned vertically higher than the position of the drain screen 60, or otherwise upstream from the position of the drain screen 60. In some embodiments, the sanitary gasket 140 may be positioned vertically lower than the position of the drain screen 60, or otherwise downstream from the position of the drain screen 60. It will further be appreciated that the outer periphery 62 of the drain screen 60 need not be molded in the inner periphery 142 of the sanitary gasket 140 and instead can be molded in an insert that is removable from the drain 40 whether by means of a tri-clamp or by other integration means. In some embodiments, the insert may be configured to rest on and fasten to an inner periphery ledge of the drain 40. In some embodiments, the drain screen 60 may form part of the drain 40 itself, for example formed in an upper portion of the port manifold 160. In still other embodiments, the outer periphery 62 of the drain screen 60 may be configured to be removable from the drain 40, that is, without being molded in an insert.

The port manifold 160 may include ports for components that aid in carrying out the processing cycle. For example, in Fig. 5, a first port 180 is configured to receive a temperature sensor, a second port 182 is configured to receive a conductivity probe, and a third port 184 is configured to receive a decontaminant fluid such as a liquid chemical sterilant or high level disinfectant solution. In some embodiments, the liquid chemical sterilant or high level disinfectant solution may comprise a solution of peracetic acid and water.

An intermediate tube 200 of the fluid line 90 connects a downstream end 164 of the port manifold 160 to an upstream end 52 of the centrifugal pump 50. The inner diameters of the intermediate tube 200 and the outlet of the downstream end 164 are smaller than (for example, about 62.5 percent) the inner diameter D1 of the drain inlet 80. The illustrated intermediate tube 200 comprises a molded pre-bent silicone tube that transitions the direction of fluid flow from along the drain axis A-A, to along an intermediate curved axis B-B, to along an axis C-C that is 90 degrees relative to the axis A-A and lies parallel to or coincides with a central axis passing through the centrifugal pump 50. Other embodiments are contemplated. For example, the intermediate tube 200 may have one or more bends to facilitate communication of the fluid from the port manifold 160 to the centrifugal pump 50. Further, the intermediate tube 200 need not transition fluid flow from axes that are oriented 90 degrees relative to one another and instead can transition the fluid flow along axes that are other than 90 degrees apart, for example, 45 degrees apart. The end connections of the intermediate tube 200 to the downstream end 164 of the port manifold 160 and to the upstream end 52 of the centrifugal pump 50 may be by barbed fittings although it will be appreciated that other means of connection may be employed.

The centrifugal pump 50 may include any type of centrifugal pump that is suitable for pumping the fluid of the centrifugal pump operated endoscope reprocessor 10 including being compatible with the fluid itself, for example, the liquid chemical sterilant or high level disinfectant solution of peracetic acid and water, as well as being compatible with the geometry of the drain screen 60. In the present embodiment, the centrifugal pump 50 is a centrifugal circulating pump. The centrifugal pump 50 is configured to draw by suction the fluid from the basin 20, through the drain 40 and the drain screen 60, and through the intermediate tube 200. The upstream end 52 of the centrifugal pump 50 includes an inlet 202 having an inner diameter that is smaller than (for example, about 62.5 percent) the inner diameter D1 of the drain inlet 80. The inner diameter of the inlet 202 translates into an area of a circle that defines a pump open area of the centrifugal pump 50. As will be described in greater detail below, the pump open area of the centrifugal pump 50 and the open area provided in the drain screen 60 preferably are compatible with one another. In some embodiments, for example, the pump open area may have about the same area as the open area provided in the drain screen 60.

The centrifugal pump 50, in turn, is connected to a manifold 210, which is shown schematically in Fig. 4. The manifold 210 is configured to direct the fluid from the centrifugal pump 50 to various parts of the centrifugal pump operated endoscope reprocessor 10 via a plurality of exit ports. In the illustrated embodiment, the manifold 210 includes a first port 220 configured to direct fluid from the centrifugal pump 50 to tubing leading to lumens and leaky port connectors of the endoscope, a second port 222 configured to direct fluid from the centrifugal pump 50 to nozzles leading to within the basin 20, a third port 224 to direct fluid from the centrifugal pump 50 to the spray mechanism 100, a fourth port 226 to direct fluid from the centrifugal pump 50 to a decontaminant fluid source that in turn leads to the basin 20 and the medical device therein, and a fifth port 228 that opens up at the conclusion of a processing cycle to funnel out the fluid from centrifugal pump operated endoscope reprocessor 10, for example, to a facility drain 240 of the health care facility.

With continued reference to Fig. 4, the centrifugal pump operated endoscope reprocessor 10 may further include a compressed air source 260 and one or more port connectors 262, five in the illustrated embodiment, that are configured to introduce compressed air into the basin 20 as fluid is drained from the basin 20 via the drain 40. In some embodiments, the compressed air source 260 may be configured to communicate air through the endoscope lumen channels and to exit the endoscope 72 through a distal tip of the endoscope 72 and/or through leaky port connectors of the endoscope 72. The leaky port connectors are leaky to enable, for example, decontamination of the endoscope port surfaces. The air released into the fluid in the basin 20 replaces the volume of the drained fluid, which aids in preventing a vacuum from forming in the basin 20.

The drain 40 is provided at the bottom of the basin 20 to allow the fluid to flow via gravity towards the drain 40. As shown in Fig. 4, a bottom wall 270 of the basin 20 has a negative slope relative to horizontal in the direction toward the bottom opening 74 in the basin 20. In the illustrated embodiment, the bottom opening 74 is positioned at the deepest part of the basin 20, that is, the vertically lowermost part of the basin 20. This enables medical devices having irregular shapes, such as the endoscope 72, to be placed in the basin 20 such that during a processing cycle the medical device is submerged in the fluid in the basin 20. As shown in Fig. 3, for example, a wider portion of the endoscope 72 such as the control section or handle 280 of the endoscope 72 can be placed in the basin 20 near the bottom opening 74 while narrower portions of the endoscope 72 such as the insertion section or tubing section 282 can be positioned in the remainder of the basin 20.

The drain 40, by being in the deepest part of the basin 20, also helps prevent air in the fluid from being pulled into the drain 40 at the drain inlet 80 by the suction created by the centrifugal pump 50. As described above, the spray mechanism 100 sprays jets of fluid down into the fluid, which may generate air in the fluid particularly in the upper part of the basin 20. However, having the drain 40 located in the deepest part of the basin 20 aids in maintaining the air generated in the upper part of the fluid in the basin 20, that is, far enough away from the drain 40 to make it possible for the air to rise to the surface of the fluid and escape the fluid.

Turning now to Fig. 6, the drain screen 60 will be described in greater detail. As noted above, the drain screen 60 is provided in the drain 40 transverse to the drain axis A-A at the depth distance D2 from the drain inlet 80, which in the illustrated embodiment is level with the sanitary gasket 140. As shown in Fig. 5, the drain screen 60 is provided in the drain 40 at a right angle, that is, perpendicular, to the drain axis A-A. Other embodiments are contemplated. For example, the drain screen 60 may be provided in the drain 40 transverse to the drain axis A-A at an angle other than a right angle to the drain axis A-A.

Also, in the illustrated embodiment, the drain screen 60 includes a perforated plate 290 which provides the afore described open area percent in the range of greater than 0 percent and up to about 70 percent of a drain area of the drain 40 at the position of the drain screen 60 in the drain 40. The perforated plate 290 may be made of any suitable material compatible with medical device processing, for example, 20-gauge stainless sheet steel. Herein, the drain screen 60 is also referred to as a two-dimensional drain screen 60 owing to the drain screen 60 being made of sheet material.

The perforated plate 290 may have any number of a plurality of perforations 292 and any size perforations 292 suitable to capture debris in the fluid passing through the drain 40. In the illustrated embodiment, the perforated plate 290 has perforations 292 having a widest width less than 6.35 mm (1/4 inch). The drain screen 60 configured with perforations 292 having a widest width less than 6.35 mm (1/4 inch) enables the drain screen 60 to prevent spore test strips that measure 6.35 mm (1/4 inch) wide x 31.75 mm (1 1/4 inch) long x 0.79 mm (1/32 inch) thick, or any other item of a reasonably small size, from entering the centrifugal pump 50. In the illustrated embodiment, the perforated plate 290 has 37 round perforations having a 4.76 mm (3/16 inch) diameter and 18 non-round perforations having a widest width of less than or equal to 4.76 mm (3/16 inch). For the round perforations, the perforation centers are 6.35 mm (1/4 inch) apart from the perforation centers of the surrounding six round perforations. In the illustrated embodiment, the perforations 292 provide the drain screen 60 with an open area percent of about 51 percent of the drain area of the drain 40 at the position of the drain screen 60 in the drain 40, which in the illustrated embodiment, as noted above, is the depth distance D2 from the drain inlet 80.

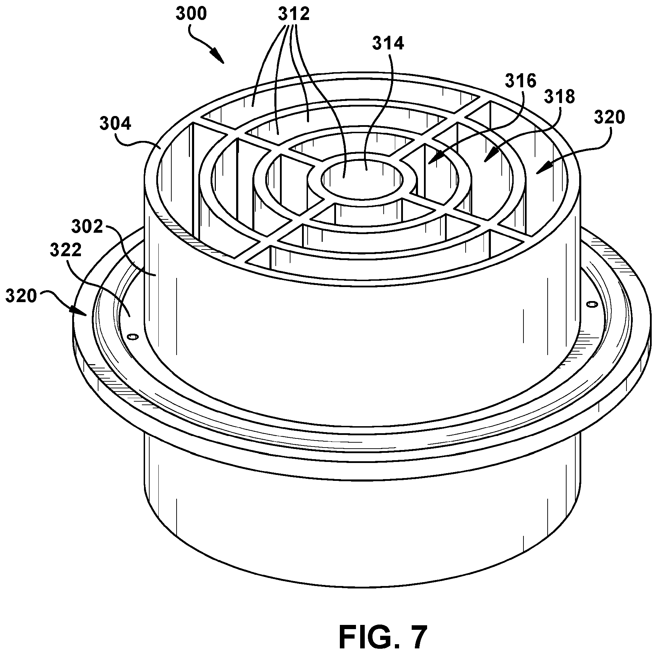

Fig. 7 shows a drain screen 300, which is an alternate embodiment of the invention. As noted above, the drain screen 300 is provided in the drain 40 transverse to the drain axis A-A at the depth distance D2 from the inlet 80 of the drain 40 in the range of about 50 percent to about 200 percent of the inner diameter D1 of the inlet 80. In some embodiments, the drain screen 300 may be provided in the drain 40 at a right angle, that is, perpendicular, to the drain axis A-A. Other embodiments are contemplated. For example, the drain screen 300 may be provided in the drain 40 transverse to the drain axis A-A at an angle other than a right angle to the drain axis A-A.

The illustrated drain screen 300 has an outer periphery 302 that is molded in or formed integrally with an inner periphery 322 of an insert 320, enabling the drain screen 300 to be coupled to the drain 40 via a tri-clamp connection, although the drain screen 300 may be connected to the drain 40 by other means, for example, as was described above with respect to the drain screen 60. The drain screen 300 is provided at the depth distance D2 from the drain inlet 80. Thus, an upper end face 304 of the drain screen 300 is at the depth distance D2 from the drain inlet 80 and level with the sanitary gasket 140 (see Fig. 5), and the drain screen 300 extends downward from the depth distance D2 to sit between the sanitary gasket 140 and the middle of the port manifold 160, it being understood that the illustrated drain screen 300 and the illustrated port manifold 160 are not to scale. In the illustrated embodiment, the drain screen 300 includes a plurality of channels 312 that provide the afore described open area percent in the range of greater than 0 percent and up to about 70 percent of the drain area of the drain 40 at the position of the drain screen 300 in the drain 40. The drain screen 300 and insert 320 may be made of any suitable material compatible with medical device processing. For example, the drain screen may be made of ABS plastic, and the insert 320 may be made of silicone rubber. Herein, the drain screen 300 is also referred to as a three-dimensional geometry drain screen 300 owing to the thickness of the drain screen 300 in the vertical direction, that is, in the direction of the drain axis A-A, being greater than a plate-like or sheet-like thickness.

The drain screen 300 may have any number of a plurality of channels 312 and any size channels 312 suitable to capture debris in the fluid passing through the drain 40. The drain screen 300 may include, for example, baffles, laminar flow tube bundles, and turbines with varying blade count and pitch. Further, the channels 312 may be configured to provide respective unimpeded flow passages from the upper end face 304 of the drain screen 300 to the middle of the port manifold 160. The unimpeded flow passages may be oriented vertically in the direction of the drain axis A-A such that the flow passages in cross section view looking down the axis A-A have a constant area. In the illustrated embodiment, the unimpeded flow passage is oriented vertically in the direction of the drain axis A-A. In the illustrated embodiment, the drain screen 300 includes a center round channel 314, and three sets 316, 318, 320 of four arc shape channels surrounding the center round channel 314 at respective first, second, and third radii. The channels 312 provide an open area percent of about 65 percent of the drain area of the drain 40 at the position of the drain screen 300 in the drain 40, which in the illustrated embodiment, as noted above, is the depth distance D2 from the drain inlet 80.

The inventor found that the problem with prior drain systems of centrifugal pump operated endoscope reprocessors where the drain screen is provided at the top surface of the drain inlet is that the drain screen does not allow enough fluid, for example decontaminant fluid, to pass through the drain if air was introduced into the fluid in the basin during a processing cycle. The inventor found that in such prior drain systems when there is a significant amount of air in the fluid in the basin, the centrifugal pump used to drain the fluid from the basin cavitates, thus preventing the fluid from being pulled by the centrifugal pump through the basin drain, or otherwise significantly reducing the flow rate of fluid pulled by the centrifugal pump.

Also, in the centrifugal pump operated endoscope reprocessor 10 where the bottom wall 270 of the basin 20 is at a negative slope relative to horizontal and the bottom opening 74 is positioned at the deepest part of the basin 20, see Fig. 4, and the spray mechanism 100 is provided, see Fig. 1, the inventor found that in the case where the drain screen is positioned at the top surface of the drain inlet 80 rather than at the depth distance D2, the jets of fluid from the spray mechanism 100 pull air into the fluid in the basin 20 as they hit the surface layer of the fluid and, although portions of the air generated in the fluid are in the upper part of the fluid in the basin 20, some of the jets of fluid increase the likelihood that air gets pulled down into the drain 40 by the centrifugal pump 50.

Also, in the centrifugal pump operated endoscope reprocessor 10 where the compressed air source 260 releases air into the basin 20 to prevent a vacuum from forming in the basin 20, or for example by communicating air via the lumen channels and distal tip and/or leaky port connectors of the endoscope 72 into the fluid in the basin 20, the inventor found that in the case where the drain screen is positioned at the top surface of the drain inlet 80 rather than at the depth distance D2, the air released into the basin 20 and/or exiting from the distal tip and/or leaky port connectors may impede the draining of the basin 20, thus resulting in increased drain time, and possibly also cavitating the centrifugal pump 50.

Also, in the centrifugal pump operated endoscope reprocessor 10 where the basin 20 is configured such that the control section or handle 280 of the endoscope 72 is placed near the bottom opening 74 of the basin 20 and the top of the drain 40, see Fig. 4, the inventor found that in the case where the drain screen is positioned at the top surface of the drain inlet 80 rather than at the depth distance D2, the air exiting the handle ports of the endoscope 72 can impede the draining of the basin 20, thus resulting in increased drain time, and possibly also cavitating the centrifugal pump 50. The inventor found that this can be particularly detrimental where the scope handle ports are located directly over the bottom opening 74 and the drain 40 since the positioning of the endoscope handle 280 in this way not only shadows the drain 40, reducing the open cross-sectional area into which fluid can flow, but also expels air directly onto the drain 40 while fluid is being pulled into the drain 40 by the centrifugal pump 50. The inventor found that the motion of the fluid from the basin 20 and into the drain 40 pulls the air down into the drain 40, and that this fluid motion dramatically increases drain time.

The inventor found that in the case where the drain screen is positioned at the top surface of the drain inlet 80 rather than at the depth distance D2, the increased drain time is due to the centrifugal pump 50 not being able to pull the fluid into the drain 40 due to the presence of air displacing volume in the drain 40 while also causing the centrifugal pump 50 to cavitate. The inventor also found that in the case where the drain screen is positioned at the top surface of the drain inlet 80 rather than at the depth distance D2, air going down the drain 40 during a gravity drain, that is draining without the aid of a centrifugal pump 50, also increased the time necessary to empty the basin 20. It is believed that this increase in drain time is due to air displacing fluid in the drain 40, reducing the available volume for fluid to flow and therefore reducing the flowrate of fluid that is pulled by the centrifugal pump through the drain.

The inventor found that the configuration of the illustrated drain 40 with no drain screen, and where air is introduced into the fluid in the basin 20 during a processing cycle, has a power failure, gravity-drain time of about five (5) minutes. The inventor also found that providing a two-dimensional geometry drain screen such as the illustrated drain screen 60 having an open area percent in the range of greater than 0 percent and up to about 70 percent and positioned at the top surface of the inlet 80 of the drain 40, that is, horizontally at the top portion of the adapter piece 120, and where air is introduced into the fluid in the basin 20 during a processing cycle, resulted in a drain time of about two (2) minutes. The inventor also found that providing a three-dimensional geometry drain screen such as the illustrated drain screen 300 having an open area percent in the range of greater than 0 percent and up to about 70 percent and positioned to extend from the top surface of the inlet 80 of the drain 40 to the middle of the port manifold 180, and where air is introduced into the fluid in the basin 20 during a processing cycle, resulted in a drain time slightly different from that of the two-dimensional geometry drain screen but no better than 10 seconds less, that is, about one (1) minute and 50 seconds.

The inventor found that positioning the drain screen, whether the two-dimensional drain screen 60 or the three-dimensional drain screen 300, at a depth distance D2 from the inlet 80 of the drain 40 in the range of about 50 percent to about 200 percent of the inner diameter D1 of the inlet 80, and where air is introduced into the fluid in the basin 20 during a processing cycle, resulted in an average drain time of 40 seconds and no cavitation in the centrifugal pump 50. By way of comparison, the drain time of 40 seconds was found to be very close to the fastest drain time of 30 seconds for the basin 20 where air was not introduced into the fluid in the basin 20 during a processing cycle. It is believed that the positioning of the drain screen 60, 300 at the depth distance D2 downward from the inlet 80 enables air in the fluid in the space above the drain screen 60, 300 to escape from the fluid or be more uniformly mixed with the fluid, enough not to cavitate the centrifugal pump 50 as the uniformly mixed fluid and air pass therethrough.

As noted above, the drain screen 60 is positioned in the drain 40 transverse to the drain axis A-A at the depth distance D2 from the drain inlet 80 of about 50 percent to about 200 percent of the inner diameter D1 of the inlet 80, which in the illustrated embodiment is at a depth distance D2 that is level with the sanitary gasket 140. Similarly, the drain screen 300 is positioned in the drain 40 transverse to the drain axis A-A at the depth distance D2 from the drain inlet 80 of about 50 percent to about 200 percent of the inner diameter D1 of the inlet 80, which in the illustrated embodiment is at a depth distance D2 in which the upper end face 304 of the drain screen 300 is level with the sanitary gasket 140. Thus, the inventor found that the geometry used for the drain screen is not the critical parameter. Rather, the critical parameter is the depth distance D2 of the drain screen 60, 300 from the inlet 80 of the drain 40.

The inventor found that the invention is realized generally by the depth distance D2 being in the range of about 50 percent to about 200 percent of the inner diameter D1 of the inlet 80, and most advantageously by a depth distance D2 of about 75 percent. In the illustrated embodiment, for example, the inner diameter D1 of the inlet 80 is about 50.8 mm (2 inches) and the depth distance D2 is about 38.1 mm (1.5 inches), that is, approximately 75 percent of the inner diameter D1. The inventor found that at less than 50 percent or greater than 200 percent, the drain screen 60, 300 does not result in mixing of the air and fluid any more effective than a drain screen positioned at the top surface of the drain inlet 80 or flush with the bottom opening 74 of the basin 20. Below 50 percent results in the introduced air blocking the available open area of the drain 40, thus reducing the amount of fluid that can get to the centrifugal pump 50, and thereby causing cavitation (similar to when the drain screen is at the top surface of the drain inlet 80 or flush with the bottom opening 74 of the basin 20). At more than 200 percent, large volumes of air are drawn into the drain 40, collect, and try to rise back out, before getting broken up by the drain screen 60, 300. The collection of air is pulled through the drain screen all at once and into the centrifugal pump 50, causing extreme cavitation.

Also, as noted above, the drain screens 60, 300 have an open area percent in the range of greater than 0 percent and up to about 70 percent of the drain area of the drain 40 at the position of the drain screen 60, 300 in the drain 40. The inventor found that the invention is realized generally by the open area percent being in the range of greater than 0 percent and up to about 70 percent. The lower number of the range, that is an open area percent greater than 0 percent, indicates the minimum for the drain screen 60, 300 to be a screen (rather than a wall). The inventor found that an open area percent that is greater than 70 percent does not break up the air that is drawn into the drain 40 enough to prevent cavitation of the centrifugal pump 50. Larger than 70 percent also does not effectively filter out particulates and/or debris such as test strips generated by a decontamination process of the centrifugal pump operated endoscope reprocessor 10.

It will be appreciated that the lower number of the drain screen open area percent range, that is greater than 0 percent, may be based on compatibility with the centrifugal pump 50. For example, the centrifugal pump 50, which in the illustrated embodiment has a pump inlet diameter of 31.75 mm (1.25 inches) has a corresponding pump open area of Pi x R^2, that is 3.14 x 15.875^2, which equates to 794 square millimetres (1.23 square inches). For a drain diameter of 50.8 mm (2 inches) at the depth distance D2, the open area percent in the range of greater than 0 percent and up to about 70 percent, results in a drain screen open area greater than zero square millimetres (zero square inches) and up to about 1413 square millimetres (2.19 square inches). As previously described, for purposes of compatibility, the pump open area of the centrifugal pump 50 may have about the same area as the open area provided in the drain screen 60. A drain screen open area percent of about 39.15 percent results in a drain screen open area of 794 square millimetres (1.23 square inches), which is most compatible with the pump open area of 794 square millimetres (1.23 square inches) of the centrifugal pump 50. For a drain screen open area percent greater than 39.15 percent (i.e., a drain screen open area greater than 794 square millimetres (1.23 square inches)) but still less than about 70 percent, the pump open area of 794 square millimetres (1.23 square inches) of the centrifugal pump 50 can easily handle the fluid pumping requirements for the drain 40 and the drain screen 60, 300 of the reprocessor 10 and without the air handling issues or cavitation issues experienced in the prior art, since the drain screen 60, 300 is at the depth distance D2 and has an open area percent in the range of greater than 0 percent and up to about 70 percent, although less compatible since a pump open area of 794 square millimetres (1.23 square inches) is lower in capacity than what is required (greater than 794 square millimetres (1.23 square inches) and up to 1413 square millimetres (2.19 square inches) for the drain 40 and the drain screen 60, 300 of the reprocessor 10. For a drain screen open area percent less than 39.15 percent (i.e., a drain screen open area less than 794 square millimetres (1.23 square inches)), the pump open area of 794 square millimetres (1.23 square inches) the centrifugal pump 50 can easily handle the fluid pumping requirements for the drain 40 and the drain screen 60, 300 of the reprocessor 10 and without the air handling issues or cavitation issues experienced in the prior art, since the drain screen 60, 300 is at the depth distance D2 and has an open area percent in the range of greater than 0 percent and up to about 70 percent, although less compatible since a pump open area of 1413 square millimetres (2.19 square inches) is more capacity than what is required (less than 794 square millimetres (1.23 square inches)) for the drain 40 and the drain screen 60, 300 of the reprocessor 10.

As will be appreciated, the geometry of the drain screen 60, 300 including the open area percent, as well as the depth distance D2 of the drain screen 60, 300, can be changed to fit other drain systems of centrifugal pump operated endoscope reprocessors 10. Further, it will be appreciated that multiple drain screens of reducing opening size may also be incorporated into the drain 40. If multiple drain screens are incorporated into the drain 40, the uppermost drain screen, that is, the drain screen closest to the inlet 80, has the depth distance D2 and has the open area percent in the range of greater than 0 percent and up to about 70 percent. The drain screens positioned downstream from the uppermost drain screen would be positioned farther down the drain 40 than the uppermost drain screen, and would have an open area percent in the range of greater than 0 percent and up to about 70 percent.

Advantageously, the drain screens 60, 300 configured in accordance with the invention enable air to be present at the drain inlet 80 without cavitating the centrifugal pump 50, resulting in a reasonable drain time. Also, advantageously, the drain screens 60, 300 configured in accordance with the invention are positioned in the drain 40 at an appropriate depth, that is, the depth distance D2, and have an appropriate open area percent, that is in the range of greater than 0 percent and up to about 70 percent, which prevents cavitation of the centrifugal pump 50 and can enable optimal drain times. Still further, advantageously, the drain screens 60, 300 configured in accordance with the invention enable the basin 20 of the centrifugal pump operated endoscope reprocessor 10 to drain with the presence of air in the fluid.

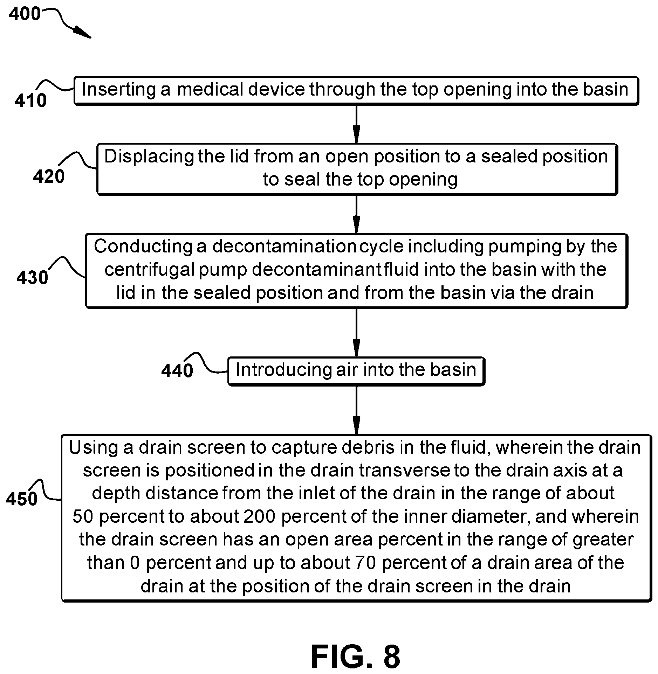

Referring now to Fig. 8, a flowchart 400 for a method of processing a medical device in a centrifugal pump operated endoscope reprocessor, such as the centrifugal pump operated endoscope reprocessor 10, is shown. Thus, the centrifugal pump operated endoscope reprocessor includes a basin having a top opening a bottom opening from which fluid exits the basin, a lid, a centrifugal pump, and a drain, the drain having an inlet at the perimeter of the bottom opening and being configured to drain fluid exiting from the bottom opening, the inlet having an inner diameter, and wherein the drain extends downward from the perimeter of the bottom opening to a fluid line leading to the centrifugal pump and the drain defines a drain axis along which the fluid flows as the centrifugal pump pumps fluid from the basin. Step 410 of the method may include inserting a medical device through the opening and into an interior of the basin. Step 420 includes displacing the lid from an open position to a sealed position to seal the top opening. Step 430 may include conducting a decontamination cycle including pumping by the centrifugal pump decontaminant fluid into the basin via one or more passageways with the lid in the sealed position and from the basin via the drain. Step 440 may include introducing air into the basin. Step 450 includes using a drain screen to capture debris in the fluid, wherein the drain screen is positioned in the drain transverse to the drain axis at a depth distance from the inlet of the drain in the range of about 50 percent to about 200 percent of the inner diameter, and wherein the drain screen has an open area percent in the range of greater than 0 percent and up to about 70 percent of a drain area of the drain at the position of the drain screen in the drain.

The method may comprise positioning a sanitary gasket in the drain at the same depth distance from the inlet of the drain as the drain screen.

The introducing air into the basin may include spraying jets of fluid within the basin.

The introducing air into the basin may include using a compressed air source to introduce air into the basin as fluid is drained from the basin via the drain.

Although the invention has been shown and described with respect to a certain embodiment or embodiments, it is obvious that equivalent alterations and modifications will occur to others skilled in the art upon the reading and understanding of this specification and the annexed drawings. In particular regard to the various functions performed by the above described elements (components, assemblies, devices, compositions, etc.), the terms (including a reference to a "means") used to describe such elements are intended to correspond, unless otherwise indicated, to any element which performs the specified function of the described element (i.e., that is functionally equivalent), even though not structurally equivalent to the disclosed structure which performs the function in the herein illustrated exemplary embodiment or embodiments of the invention. In addition, while a particular feature of the invention may have been described above with respect to only one or more of several illustrated embodiments, such feature may be combined with one or more other features of the other embodiments, as may be desired and advantageous for any given or particular application.

## Claims

1. A centrifugal pump operated endoscope reprocessor, (10) comprising:
a basin (20) having a top opening (70) for receiving therein a medical device (72) for processing and a bottom opening (74) from which fluid exits the basin (20);
a lid (30) displaceable relative to the basin (20) between an open position to expose the top opening (70) and a sealed position to seal the top opening (70);
a drain (40) having an inlet (80) at the perimeter of the bottom opening (74) of the basin (20) and being configured to drain fluid exiting from the bottom opening (74), wherein the inlet (80) has an inner diameter;
a centrifugal pump (50) configured to pump fluid to the basin (20) via one or more passageways (220,222,224) with the lid (30) in the sealed position and from the basin (20) via the drain (40), wherein the drain (40) extends downward from the perimeter of the bottom opening (74) of the basin to the fluid line (90) leading to the centrifugal pump (50), wherein the drain (40) defines a drain axis along which the fluid flows as the centrifugal pump (50) pumps fluid from the basin (20); and,
a drain screen (60,300) configured to capture debris in the fluid, wherein the drain screen (60,300) is positioned in the drain (40) transverse to the drain axis at a depth distance from the inlet (80) of the drain in the range of 50 percent to 200 percent of the inner diameter, and
wherein the drain screen (60,300) has an open area percent in the range of greater than 0 percent and up to about 70 percent of a drain area of the drain (40) at the position of the drain screen (60,300) in the drain (40).

2. The centrifugal pump operated endoscope reprocessor of claim 1, wherein the depth distance is 75 percent of the inner diameter.

3. The centrifugal pump operated endoscope reprocessor of any of claims 1-2, wherein the centrifugal pump (50) has a pump open area that is the same area as the open area of the drain screen (60,300).

4. The centrifugal pump operated endoscope reprocessor of any of claims 1-3, wherein the drain screen (60,300) includes a perforated plate.

5. The centrifugal pump operated endoscope reprocessor of claim 4, wherein the perforated plate has a plurality of perforations having a widest width less than 6.35mm (1/4 inch).

6. The centrifugal pump operated endoscope reprocessor of claim 5, wherein the widest width of the perforations is less than or equal to 4.76mm ((3/16 inch).

7. The centrifugal pump operated endoscope reprocessor of any of claims 1-6, wherein the drain screen (60,300) comprises an insert removable from the drain.

8. The centrifugal pump operated endoscope reprocessor of any of claims 1-7, comprising a sanitary gasket (140).

9. The centrifugal pump operated endoscope reprocessor of claim 8, wherein the sanitary gasket (140) is positioned in the drain (40) at the same depth distance from the inlet (80) of the drain as the drain screen (60,300).

10. The centrifugal pump operated endoscope reprocessor of claim 9, wherein an outer periphery of the drain screen (60,300) is molded in an inner periphery of the sanitary gasket (140).

11. The centrifugal pump operated endoscope reprocessor of any of claims 1-10, wherein a bottom wall (270) of the basin (20) has a negative slope relative to horizontal in the direction toward the bottom opening (74), and the bottom opening (74) is positioned at the deepest part of the basin (20).

12. The centrifugal pump operated endoscope reprocessor of any of claims 1-11, comprising a spray mechanism (100) configured to spray jets of fluid within the basin (20).

13. The centrifugal pump operated endoscope reprocessor of claim 12, wherein the spray mechanism (100) includes a spray bar (102) rotatably mounted to an underside of the lid (30).

14. The centrifugal pump operated endoscope reprocessor of any of claims 1-13, comprising a compressed air source (260) configured to introduce compressed air into the basin as fluid is drained from the basin (20) via the drain (40).

15. A method of processing a medical device in a centrifugal pump operated endoscope reprocessor including a basin (20) having a top opening (70), a bottom opening (74) from which fluid exits the basin, a lid (30), a centrifugal pump (50), and a drain (40), the drain (40) having an inlet (80) at the perimeter of the bottom opening (74) and being configured to drain fluid exiting from the bottom opening (74), the inlet (80) having an inner diameter, and wherein the drain (40) extends downward from the perimeter of the bottom opening (74) to a fluid line (90) leading to the centrifugal pump (50) and the drain (40) defines a drain axis along which the fluid flows as the centrifugal pump (50) pumps fluid from the basin (20), the method comprising:
inserting a medical device through the top opening (70) into the basin (20);
displacing the lid (30) from an open position to a sealed position to seal the top opening (70);
conducting a decontamination cycle including pumping by the centrifugal pump decontaminant fluid into the basin (20) via one or more passageways (220,222,224) with the lid (30) in the sealed position and from the basin (20) via the drain (40);
introducing air into the basin (20);
using a drain screen (60,300) to capture debris in the fluid, wherein the drain screen (60,300) is positioned in the drain (40) transverse to the drain axis at a depth distance from the inlet (80) of the drain (40) in the range of 50 percent to 200 percent of the inner diameter, and wherein the drain screen (60,300) has an open area percent in the range of greater than 0 percent and up to 70 percent of a drain area of the drain (40) at the position of the drain screen (60,300) in the drain (40).

16. The method of claim 15, comprising positioning a sanitary gasket in the drain (40) at the same depth distance from the inlet (80) of the drain (40) as the drain screen (60,300).

17. The method of any of claims 15-16, wherein the introducing air into the basin (20) includes spraying jets of fluid within the basin (20).

18. The method of any of claims 15-17, wherein the introducing air into the basin includes using a compressed air source (260) to introduce air into the basin (20) as fluid is drained from the basin (20) via the drain (40).

19. The method of any of claims 15-18, wherein the inserting the medical device includes inserting an endoscope (72) such that a handle of the endoscope is positioned at the surface of the drain inlet (80).

## Patentansprüche

1. Zentrifugalpumpenbetriebener Endoskopreprozessor, (10) umfassend:
ein Becken (20) mit einer oberen Öffnung (70) zur Aufnahme einer aufzubereitenden medizinischen Vorrichtung (72) darin und einer unteren Öffnung (74), aus der Flüssigkeit aus dem Becken (20) austritt;
einen Deckel (30), der relativ zum Becken (20) zwischen einer offenen Position, um die obere Öffnung (70) freizulegen, und einer abgedichteten Position, um die obere Öffnung (70) abzudichten, verschiebbar ist;
einen Abfluss (40), der einen Einlass (80) an dem Umfang der unteren Öffnung (74) des Beckens (20) aufweist und der dazu konfiguriert ist, aus der unteren Öffnung (74) austretende Flüssigkeit abzuleiten, wobei der Einlass (80) einen Innendurchmesser aufweist;
eine Zentrifugalpumpe (50), die dazu konfiguriert ist, Flüssigkeit über einen oder mehrere Kanäle (220,222,224) in das Becken (20) zu pumpen, wenn sich der Deckel (30) in der abgedichteten Position befindet, und über den Abfluss (40) aus dem Becken (20) zu pumpen, wobei sich der Abfluss (40) von dem Umfang der unteren Öffnung (74) des Beckens nach unten zu der Flüssigkeitsleitung (90) erstreckt, die zur Zentrifugalpumpe (50) führt, wobei der Abfluss (40) eine Abflussachse definiert, entlang der die Flüssigkeit fließt, während die Zentrifugalpumpe (50) Flüssigkeit aus dem Becken (20) pumpt; und
ein Abflusssieb (60,300), das dazu konfiguriert ist, Verunreinigungen in der Flüssigkeit aufzufangen, wobei das Abflusssieb (60,300) in dem Abfluss (40) quer zu der Abflussachse in einem Tiefenabstand vom Einlass (80) des Abflusses im Bereich von 50 Prozent bis 200 Prozent des Innendurchmessers positioniert ist und
wobei das Abflusssieb (60,300) einen offenen Flächenanteil im Bereich von mehr als 0 Prozent bis zu etwa 70 Prozent einer Abflussfläche des Abflusses (40) an der Position des Abflusssiebs (60,300) in dem Abfluss (40) aufweist.

2. Zentrifugalpumpenbetriebener Endoskopreprozessor nach Anspruch 1, wobei der Tiefenabstand 75 Prozent des Innendurchmessers beträgt.

3. Zentrifugalpumpenbetriebener Endoskopreprozessor nach einem der Ansprüche 1 bis 2, wobei die Zentrifugalpumpe (50) eine offene Pumpenfläche aufweist, die derselben Fläche entspricht wie die offene Fläche des Abflusssiebs (60,300).

4. Zentrifugalpumpenbetriebener Endoskopreprozessor nach einem der Ansprüche 1 bis 3, wobei das Abflusssieb (60,300) eine perforierte Platte beinhaltet.

5. Zentrifugalpumpenbetriebener Endoskopreprozessor nach Anspruch 4, wobei die perforierte Platte eine Vielzahl von Perforationen mit einer größten Breite von weniger als 6,35 mm (1/4 Zoll) aufweist.

6. Zentrifugalpumpenbetriebener Endoskopreprozessor nach Anspruch 5, wobei die größte Breite der Perforationen kleiner als oder gleich 4,76 mm ((3/16 Zoll) ist.

7. Zentrifugalpumpenbetriebener Endoskopreprozessor nach einem der Ansprüche 1 bis 6, wobei das Abflusssieb (60,300) einen aus dem Abfluss entnehmbaren Einsatz umfasst.

8. Zentrifugalpumpenbetriebener Endoskopreprozessor nach einem der Ansprüche 1 bis 7, umfassend eine hygienische Dichtung (140).

9. Zentrifugalpumpenbetriebener Endoskopreprozessor nach Anspruch 8, wobei die hygienische Dichtung (140) in dem Abfluss (40) in demselben Tiefenabstand von dem Einlass (80) des Abflusses positioniert ist wie das Abflusssieb (60,300).

10. Zentrifugalpumpenbetriebener Endoskopreprozessor nach Anspruch 9, wobei ein äußerer Umfang des Abflusssiebs (60,300) in einen inneren Umfang der hygienischen Dichtung (140) eingeformt ist.

11. Zentrifugalpumpenbetriebener Endoskopreprozessor nach einem der Ansprüche 1 bis 10, wobei eine Bodenwand (270) des Beckens (20) ein negatives Gefälle relativ zu der Horizontalen in Richtung der unteren Öffnung (74) aufweist und die untere Öffnung (74) am tiefsten Teil des Beckens (20) positioniert ist.

12. Zentrifugalpumpenbetriebener Endoskopreprozessor nach einem der Ansprüche 1 bis 11, umfassend einen Sprühmechanismus (100), der dazu konfiguriert ist, Flüssigkeitsstrahlen innerhalb des Beckens (20) zu versprühen.

13. Zentrifugalpumpenbetriebener Endoskopreprozessor nach Anspruch 12, wobei der Sprühmechanismus (100) einen Sprühbalken (102) beinhaltet, der drehbar an einer Unterseite des Deckels (30) angebracht ist.

14. Zentrifugalpumpenbetriebener Endoskopreprozessor nach einem der Ansprüche 1 bis 13, umfassend eine Druckluftquelle (260), die dazu konfiguriert ist, Druckluft in das Becken einzuleiten, während Flüssigkeit über den Abfluss (40) aus dem Becken (20) abgeleitet wird.

15. Verfahren zur Aufbereitung einer medizinischen Vorrichtung in einem zentrifugalpumpenbetriebenen Endoskopreprozessor, der ein Becken (20) mit einer oberen Öffnung (70), einer unteren Öffnung (74), aus der Flüssigkeit aus dem Becken austritt, einen Deckel (30), eine Zentrifugalpumpe (50) und einen Abfluss (40) beinhaltet, wobei der Abfluss (40) einen Einlass (80) an dem Umfang der unteren Öffnung (74) aufweist und dazu konfiguriert ist, aus der unteren Öffnung (74) austretende Flüssigkeit abzuleiten, wobei der Einlass (80) einen Innendurchmesser aufweist und wobei sich der Abfluss (40) von dem Umfang der unteren Öffnung (74) nach unten zu einer Flüssigkeitsleitung (90) erstreckt, die zu der Zentrifugalpumpe (50) führt, und der Abfluss (40) eine Abflussachse definiert, entlang der die Flüssigkeit fließt, während die Zentrifugalpumpe (50) Flüssigkeit aus dem Becken (20) pumpt, wobei das Verfahren umfasst:
Einführen einer medizinischen Vorrichtung durch die obere Öffnung (70) in das Becken (20);
Verschieben des Deckels (30) von einer offenen Position in eine abgedichtete Position, um die obere Öffnung (70) abzudichten;
Durchführen eines Dekontaminationszyklus, der das Pumpen von Dekontaminationsflüssigkeit durch die Zentrifugalpumpe in das Becken (20) über einen oder mehrere Kanäle (220,222,224), wenn sich der Deckel (30) in der abgedichteten Position befindet, und aus dem Becken (20) über den Abfluss (40) beinhaltet;
Einleiten von Luft in das Becken (20);
Verwenden eines Abflusssiebs (60,300) zum Auffangen von Verunreinigungen in der Flüssigkeit, wobei das Abflusssieb (60,300) in dem Abfluss (40) quer zu der Abflussachse in einem Tiefenabstand von dem Einlass (80) des Abflusses (40) im Bereich von 50 Prozent bis 200 Prozent des Innendurchmessers positioniert ist und wobei das Abflusssieb (60,300) einen offenen Flächenanteil im Bereich von mehr als 0 Prozent bis zu 70 Prozent einer Abflussfläche des Abflusses (40) an der Position des Abflusssiebs (60,300) im Abfluss (40) aufweist.

16. Verfahren nach Anspruch 15, umfassend das Positionieren einer hygienischen Dichtung in dem Abfluss (40) in demselben Tiefenabstand von dem Einlass (80) des Abflusses (40) wie das Abflusssieb (60,300).

17. Verfahren nach einem der Ansprüche 15 bis 16, wobei das Einleiten von Luft in das Becken (20) das Versprühen von Flüssigkeitsstrahlen innerhalb des Beckens (20) beinhaltet.

18. Verfahren nach einem der Ansprüche 15 bis 17, wobei das Einleiten von Luft in das Becken die Verwendung einer Druckluftquelle (260) beinhaltet, um Luft in das Becken (20) einzuleiten, während Flüssigkeit über den Abfluss (40) aus dem Becken (20) abgeleitet wird.

19. Verfahren nach einem der Ansprüche 15 bis 18, wobei das Einführen der medizinischen Vorrichtung das Einführen eines Endoskops (72) beinhaltet, sodass ein Griff des Endoskops an der Oberfläche des Abflusseinlasses (80) positioniert ist.

## Revendications

1. Dispositif de retraitement d'endoscope actionné par pompe centrifuge (10) comprenant :
un bassin (20) ayant une ouverture supérieure (70) destinée à recevoir en son sein un dispositif médical (72) pour traitement et une ouverture inférieure (74) par laquelle le fluide sort du bassin (20) ;
un couvercle (30) déplaçable par rapport au bassin (20) entre une position ouverte pour exposer l'ouverture supérieure (70) et une position scellée pour sceller l'ouverture supérieure (70) ;
un drain (40) ayant une entrée (80) au niveau du périmètre de l'ouverture inférieure (74) du bassin (20) et étant configuré pour drainer le fluide sortant de l'ouverture inférieure (74), dans lequel l'entrée (80) a un diamètre intérieur ;
une pompe centrifuge (50) configurée pour pomper le fluide vers le bassin (20) via un ou plusieurs passages (220, 222, 224) lorsque le couvercle (30) est en position scellée, et depuis le bassin (20) via le drain (40), dans lequel le drain (40) s'étend vers le bas depuis le périmètre de l'ouverture inférieure (74) du bassin jusqu'à la conduite de fluide (90) menant à la pompe centrifuge (50), dans lequel le drain (40) définit un axe de drain le long duquel le fluide s'écoule à mesure que la pompe centrifuge (50) pompe le fluide depuis le bassin (20) ; et,
un tamis de drainage (60, 300) configuré pour capturer des débris dans le fluide, dans lequel le tamis de drainage (60, 300) est positionné dans le drain (40) transversalement à l'axe de drain à une distance de profondeur depuis l'entrée (80) du drain comprise dans la plage allant de 50 pour cent à 200 pour cent du diamètre intérieur, et
dans lequel le tamis de drainage (60, 300) a un pourcentage de zone d'ouverture compris dans la plage allant de plus de 0 pour cent et jusqu'à 70 pour cent d'une zone de drain du drain (40) au niveau de la position du tamis de drainage (60, 300) dans le drain (40).

2. Dispositif de retraitement d'endoscope actionné par pompe centrifuge selon la revendication 1, dans lequel la distance de profondeur est de 75 pour cent du diamètre intérieur.

3. Dispositif de retraitement d'endoscope actionné par pompe centrifuge selon l'une quelconque des revendications 1 et 2, dans lequel la pompe centrifuge (50) a une zone d'ouverture de pompe qui est la même zone que la zone d'ouverture du tamis de drainage (60, 300).

4. Dispositif de retraitement d'endoscope actionné par pompe centrifuge selon l'une quelconque des revendications 1 à 3, dans lequel le tamis de drainage (60, 300) comporte une plaque perforée.

5. Dispositif de retraitement d'endoscope actionné par pompe centrifuge selon la revendication 4, dans lequel la plaque perforée a une pluralité de perforations ayant une largeur maximale inférieure à 6,35 mm (1/4 pouce).

6. Dispositif de retraitement d'endoscope actionné par pompe centrifuge selon la revendication 5, dans lequel la largeur maximale des perforations est inférieure ou égale à 4,76 mm (3/16 pouce).

7. Dispositif de retraitement d'endoscope actionné par pompe centrifuge selon l'une quelconque des revendications 1 à 6, dans lequel le tamis de drainage (60, 300) comprend un insert amovible du drain.

8. Dispositif de retraitement d'endoscope actionné par pompe centrifuge selon l'une quelconque des revendications 1 à 7, comprenant un joint sanitaire (140).

9. Dispositif de retraitement d'endoscope actionné par pompe centrifuge selon la revendication 8, dans lequel le joint sanitaire (140) est positionné dans le drain (40) à la même distance de profondeur depuis l'entrée (80) du drain que le tamis de drainage (60, 300).

10. Dispositif de retraitement d'endoscope actionné par pompe centrifuge selon la revendication 9, dans lequel une périphérie extérieure du tamis de drainage (60,300) est moulée dans une périphérie intérieure du joint sanitaire (140).

11. Dispositif de retraitement d'endoscope actionné par pompe centrifuge selon l'une quelconque des revendications 1 à 10, dans lequel une paroi inférieure (270) du bassin (20) a une pente négative par rapport à l'horizontale dans la direction vers l'ouverture inférieure (74), et l'ouverture inférieure (74) est positionnée au niveau de la partie la plus profonde du bassin (20).

12. Dispositif de retraitement d'endoscope actionné par pompe centrifuge selon l'une quelconque des revendications 1 à 11, comprenant un mécanisme de pulvérisation (100) configuré pour pulvériser des jets de fluide à l'intérieur du bassin (20).

13. Dispositif de retraitement d'endoscope actionné par pompe centrifuge selon la revendication 12, dans lequel le mécanisme de pulvérisation (100) comporte une barre de pulvérisation (102) montée de manière rotative sur une face inférieure du couvercle (30).

14. Dispositif de retraitement d'endoscope actionné par pompe centrifuge selon l'une quelconque des revendications 1 à 13, comprenant une source d'air comprimé (260) configurée pour introduire de l'air comprimé dans le bassin à mesure que le fluide est drainé depuis le bassin (20) via le drain (40).

15. Procédé de traitement d'un dispositif médical dans un dispositif de retraitement d'endoscope actionné par pompe centrifuge comportant un bassin (20) ayant une ouverture supérieure (70), une ouverture inférieure (74) par laquelle le fluide sort du bassin, un couvercle (30), une pompe centrifuge (50) et un drain (40), le drain (40) ayant une entrée (80) au niveau du périmètre de l'ouverture inférieure (74) et étant configuré pour drainer le fluide sortant de l'ouverture inférieure (74), l'entrée (80) ayant un diamètre intérieur, et dans lequel le drain (40) s'étend vers le bas depuis le périmètre de l'ouverture inférieure (74) jusqu'à une conduite de fluide (90) menant à la pompe centrifuge (50), et le drain (40) définit un axe de drain le long duquel le fluide s'écoule à mesure que la pompe centrifuge (50) pompe le fluide depuis le bassin (20), le procédé comprenant :
l'insertion d'un dispositif médical à travers l'ouverture supérieure (70) dans le bassin (20) ;
le déplacement du couvercle (30) d'une position ouverte à une position scellée pour sceller l'ouverture supérieure (70) ;
la mise en œuvre d'un cycle de décontamination comportant le pompage, par la pompe centrifuge, du fluide décontaminant dans le bassin (20) via un ou plusieurs passages (220, 222, 224) lorsque le couvercle (30) est en position scellée, et depuis le bassin (20) via le drain (40) ;
l'introduction d'air dans le bassin (20) ;
l'utilisation d'un tamis de drainage (60, 300) pour capturer des débris dans le fluide, dans lequel le tamis de drainage (60, 300) est positionné dans le drain (40) transversalement à l'axe de drain à une distance de profondeur depuis l'entrée (80) du drain (40) comprise dans la plage allant de 50 pour cent à 200 pour cent du diamètre intérieur, et dans lequel le tamis de drainage (60, 300) a un pourcentage de zone d'ouverture compris dans la plage allant de plus de 0 pour cent et jusqu'à 70 pour cent d'une zone de drain du drain (40) au niveau de la position du tamis de drainage (60, 300) dans le drain (40).

16. Procédé selon la revendication 15, comprenant le positionnement d'un joint sanitaire dans le drain (40) à la même distance de profondeur depuis l'entrée (80) du drain (40) que le tamis de drainage (60, 300).

17. Procédé selon l'une quelconque des revendications 15 et 16, dans lequel l'introduction d'air dans le bassin (20) comporte la pulvérisation de jets de fluide à l'intérieur du bassin (20).

18. Procédé selon l'une quelconque des revendications 15 à 17, dans lequel l'introduction d'air dans le bassin comporte l'utilisation d'une source d'air comprimé (260) pour introduire de l'air dans le bassin (20) à mesure que le fluide est drainé depuis le bassin (20) via le drain (40).

19. Procédé selon l'une quelconque des revendications 15 à 18, dans lequel l'insertion du dispositif médical comporte l'insertion d'un endoscope (72) de telle sorte qu'une poignée de l'endoscope soit positionnée au niveau de la surface de l'entrée (80) de drain.
